# EUROPEAN PATENT APPLICATION

(11) **EP 4 589 329 A2**
(43) Date of publication of application: **23.07.2025**
(21) Application number: 24222377.4
(22) Date of filing: 20.12.2024
(51) Int. Cl.: G01S 7/00, G01S 7/02, G01S 13/04, G01S 13/42, G01S 13/56, G01S 13/88, G01S 13/02

(54) **INDOOR RADAR DEVICE, INDOOR EVENT DETECTING SYSTEM AND METHOD THEREOF**

(30) Priority: 29.12.2023 US 202363615798 P; 08.08.2024 TW 113129730
(71) Applicant: Cubtek Inc., Zhubei City, Hsinchu County 302 (TW)
(72) Inventor: HUNG, Yuan-Tung, Zhubei City, Hsinchu County (TW); HUANG, Kuan-Kai, Zhubei City, Hsinchu County (TW); LEE, Kou-Ting, Zhubei City, Hsinchu County (TW); SYU, Jia-Hao, Zhubei City, Hsinchu County (TW); FU, Chuan-Ting, Zhubei City, Hsinchu County (TW); SHIH, Wei-Shun, Zhubei City, Hsinchu County (TW)
(74) Representative: Vossius & Partner Patentanwälte Rechtsanwälte mbB

(57) **Abstract**

An indoor radar device includes a housing outer surface, which includes at least one mounting surface and an upper inclined surface. The mounting surface is configured to be connected to an indoor surface of a room. The upper inclined surface is configured to inclinedly face a ceiling of the room. An upper inclined angle between the upper inclined surface and a floor of the room is between 15 degrees and 85 degrees.

## Description

### BACKGROUND

### Technical Field

The present disclosure relates to an indoor radar device, an indoor event detecting system and a method thereof. More particularly, the present disclosure relates to an indoor radar device, an indoor event detecting system and a method thereof for the conditions that are not suitable for using image detection.

### Description of Related Art

When deaths occur in confined spaces such as public toilets, most cases are due to sudden dizziness, heart failure and other factors. This is due to failure to detect in time in the confined space, so the golden rescue time is missed.

Given the above, for the confined spaces that are not suitable for image detection, such as toilet rooms, bathrooms, or dressing rooms, there is an urgent need to develop a device, system and method that can detect events or health conditions of people in the confined spaces to avoid regrets.

### SUMMARY

According to one aspect of the present disclosure, an indoor radar device includes a housing outer surface, which includes at least one mounting surface and an upper inclined surface. The mounting surface is configured to be connected to an indoor surface of a room. The upper inclined surface is configured to inclinedly face a ceiling of the room. An upper inclined angle between the upper inclined surface and a floor of the room is between 15 degrees and 85 degrees.

According to the indoor radar device of the foregoing aspect, the upper inclined surface may be a convex arc surface and has a plurality of normal directions. An average surface roughness of the upper inclined surface is less than 5 mm. A contact angle of the upper inclined surface is greater than 30 degrees. The indoor surface is a wall surface of the room or a surface in the room being not parallel to the floor.

According to the indoor radar device of the foregoing aspect, the indoor radar device may further include a first antenna array including at least two antennas. The two antennas are arranged along a direction from the floor to the ceiling, a distance between the two antennas may be 0.5 times a wavelength, and the wavelength may be between 0.001 mm and 12 mm.

According to the indoor radar device of the foregoing aspect, the housing outer surface may further include a first emitting surface. The first antenna array is adjacent to or disposed on the first emitting surface, the first emitting surface is configured to be closer to the floor than the upper inclined surface thereto, the first emitting surface is configured to inclinedly face the floor, and a first angle between the first emitting surface and the mounting surface may be between 5 degrees and 75 degrees.

According to the indoor radar device of the foregoing aspect, the indoor radar device may further include a second antenna array. The housing outer surface further includes a second emitting surface. The second antenna array is adjacent to or disposed on the second emitting surface, the second emitting surface is configured to inclinedly face the floor, and a second angle between the first emitting surface and the second emitting surface may be between 105 degrees and 170 degrees.

According to the indoor radar device of the foregoing aspect, a number of the at least one mounting surface may be two, and a mounting surface angle between the two mounting surfaces may be between 70 degrees and 110 degrees. The upper inclined surface is connected between the two mounting surfaces, and the upper inclined surface is configured to be taped along a direction from the floor to the ceiling.

According to the indoor radar device of the foregoing aspect, the indoor radar device may include a radar unit and an emergency notification unit. The emergency notification unit is configured to be triggered by a detected target in the room to notify an emergency state, and the emergency notification unit is configured to be connected to a part of the radar unit away from the floor. A radar outer surface of the housing outer surface corresponds to the radar unit. An emergency notification outer surface of the housing outer surface corresponds to the emergency notification unit, and the upper inclined surface is located on the emergency notification outer surface.

According to the indoor radar device of the foregoing aspect, the radar unit may include a first antenna array, and the radar outer surface includes a first emitting surface. The first antenna array is adjacent to or disposed on the first emitting surface, and the first emitting surface is configured to be located at an extension direction from the upper inclined surface to the floor.

According to another aspect of the present disclosure, an indoor radar device is configured to be connected to an indoor surface of a room and includes a radar unit, an emergency notification unit and a housing outer surface. The radar unit includes a first antenna array, which includes at least two antennas. The two antennas are arranged along a direction from a floor to a ceiling of the room. A distance between the two antennas may be 0.5 times a wavelength (i.e., one half of a wavelength), and the wavelength may be between 0.001 mm and 12 mm. The emergency notification unit coupled to the radar unit. The housing outer surface includes a radar outer surface and an emergency notification outer surface. The radar outer surface corresponds to the radar unit, and the emergency notification outer surface corresponds to the emergency notification unit and is configured to be triggered by a detected target in the room to notify an emergency state.

According to the indoor radar device of the foregoing aspect, the emergency notification unit may be configured to be connected to a part of the radar unit away from the floor.

According to the indoor radar device of the foregoing aspect, the radar outer surface may include a first emitting surface, the first antenna array is adjacent to or disposed on the first emitting surface, and the first emitting surface is perpendicular to the floor.

According to the indoor radar device of the foregoing aspect, the emergency notification outer surface may include an upper inclined surface, which is configured to inclinedly face a ceiling of the room, and the first emitting surface is configured to be closer to the floor than the upper inclined surface thereto.

According to the indoor radar device of the foregoing aspect, the housing outer surface may further include two mounting surfaces, which are configured to be connected to the indoor surface, and a mounting surface angle between the two mounting surfaces may be between 70 degrees and 110 degrees.

According to the indoor radar device of the foregoing aspect, the housing outer surface may further include at least one mounting surface, which is configured to be connected to the indoor surface. The housing outer surface includes a first emitting surface. The first antenna array is adjacent to or disposed on the first emitting surface, the first emitting surface is configured to inclinedly face the floor, and a first angle between the first emitting surface and the mounting surface is between 5 degrees and 75 degrees.

According to the indoor radar device of the foregoing aspect, the indoor radar device may be configured for detecting whether the detected target satisfies a fall condition, and a distance between the indoor radar device and the floor may be less than or equal to 90 cm.

According to another aspect of the present disclosure, an indoor event detecting system includes at least one indoor radar device, at least one door-closed sensing device and a control center. The at least one indoor radar device is configured to be disposed on at least one indoor surface of at least one room and includes at least one housing outer surface, which includes an upper inclined surface. The at least one door-closed sensing device is configured to be disposed in the at least one room. The control center is wiredly or wirelessly communicatively connected to the at least one indoor radar device and the at least one door-closed sensing device. The indoor event detecting system is configured to: detect whether a door of the room starts to be in a door-closed state operated inside the room by the door-closed sensing device; perform an occupancy detection to determine whether there is a detected target in the room and determine whether an overtime occupancy condition is satisfied by the indoor radar device when it is detected that the door starts to be in the door-closed state; perform a vital sign detection to determine whether a dangerous condition is satisfied by the indoor radar device when it is detected that the door starts to be in the door-closed state; and determine that an indoor dangerous event exists in the room and generate an alarm when at least one of the overtime occupancy condition and the dangerous condition is satisfied.

According to the indoor event detecting system of the foregoing aspect, the room may be one of a toilet room, a bathroom and a dressing room.

According to the indoor event detecting system of the foregoing aspect, the indoor surface may be a wall surface of the room or a surface in the room being not parallel to a floor. The indoor radar device may further include a first antenna array, and the housing outer surface may further include a first emitting surface. The first antenna array is adjacent to or disposed on the first emitting surface, the first emitting surface is configured to face a heart of the detected target, and a distance between the indoor radar device and the floor is greater than 70 cm.

According to the indoor event detecting system of the foregoing aspect, the door may include a driving side and an open side. The door-closed sensing device includes a first sensing element and a second sensing element. The first sensing element is configured to be disposed on an upper end of the open side, the second sensing element is configured to be disposed on the indoor surface, and the first sensing element and the second sensing element are configured to detect a distance between each other. When the door is in the door-closed state, the first sensing element and the second sensing element are disposed adjacent to each other.

According to the indoor event detecting system of the foregoing aspect, a number of the at least one indoor surface may be two. The two indoor surfaces form a corner, the indoor radar device is disposed on the corner and further includes a radar unit and an emergency notification unit, which is configured to be triggered by a detected target in the room to notify an emergency state, and the emergency notification unit is configured to be connected to a part of the radar unit away from a floor.

According to the indoor event detecting system of the foregoing aspect, the door may include a driving side and an open side, and a door lock assembly of the room includes a first lock element and a second lock element. The first lock element is disposed on the open side, and the second lock element is disposed on the indoor surface. The door-closed sensing device is configured to be disposed at at least one of the first lock element and the second lock element to detect whether the door is in the door-closed state.

According to another aspect of the present disclosure, an indoor event detecting method includes: detecting whether a door of a room starts to be in a door-closed state operated inside the room by a door-closed sensing device disposed in the room; performing an occupancy detection to determine whether there is a detected target in the room and determine whether an overtime occupancy condition is satisfied by an indoor radar device when it is detected that the door starts to be in the door-closed state, wherein the indoor radar device includes a housing outer surface, which includes an upper inclined surface; performing a vital sign detection to determine whether a dangerous condition is satisfied by the indoor radar device when it is detected that the door starts to be in the door-closed state; and determining that an indoor dangerous event exists in the room and generate an alarm when at least one of the overtime occupancy condition and the dangerous condition is satisfied.

According to the indoor event detecting method of the foregoing aspect, the indoor event detecting method may further include communicatively pairing the indoor radar device and a control center, and the indoor radar device and the control center are wirelessly connected via at least one of a frequency division multiplexing manner and a time division multiplexing manner.

According to the indoor event detecting method of the foregoing aspect, the room may be a toilet room and divided into a door-closed detection space and an occupancy detection space, the door-closed detection space is adjacent to the door, and the occupancy detection space is adjacent to a toilet in the room. The indoor event detecting method may further include detecting whether the door starts to be in the door-closed state operated inside the room by the indoor radar device, which includes detecting whether the detected target moves from the door-closed detection space to the occupancy detection space.

According to the indoor event detecting method of the foregoing aspect, the overtime occupancy condition may include that an occupancy time value of the detected target in the room is greater than an occupancy time threshold, which is between 3 minutes and 90 minutes.

According to the indoor event detecting method of the foregoing aspect, performing the vital sign detection may include at least one of performing a posture detection, performing a heart rate detection and performing a respiration detection. Performing the posture detection includes detecting whether a detected target stationary condition is satisfied and whether a fall condition is satisfied based on at least five sampling points by the indoor radar device, which is a posture detection result. Performing the heart rate detection includes detecting a heart rate related to a time to determine whether an abnormal heart rate condition is satisfied, which is a heart rate detection result. Performing the respiration detection includes detecting a respiration rate related to the time to determine whether an abnormal respiration condition is satisfied, which is a respiration detection result. The indoor event detecting method may further include sending the heart rate and the respiration rate to a control center by the indoor radar device and storing the heart rate and the respiration rate in the control center; and determining whether the dangerous condition is satisfied based on corresponding at least one of the posture detection result, the heart rate detection result and the respiration detection result.

### BRIEF DESCRIPTION OF THE DRAWINGS

The present disclosure can be more fully understood by reading the following detailed description of the embodiment, with reference made to the accompanying drawings as follows:
Fig. 1A is a three-dimensional view of an indoor radar device according to the first embodiment of the present disclosure.
Fig. 1B is a top view of the indoor radar device in Fig. 1A.
Fig. 1C is a cross-sectional view along line 1C-1C of the indoor radar device in Fig. 1B.
Fig. 1D is a schematic view of a first antenna array of the indoor radar device in Fig. 1C.
Fig. 2A is a three-dimensional view of an indoor radar device according to the second embodiment of the present disclosure.
Fig. 2B is a top view of the indoor radar device in Fig. 2A.
Fig. 2C is a cross-sectional view along line 2C-2C of the indoor radar device in Fig. 2B.
Fig. 3A is a schematic view of an indoor event detecting system according to the third embodiment of the present disclosure.
Fig. 3B is a block diagram of the indoor event detecting system in Fig. 3A.
Fig. 4 is a schematic view of an indoor event detecting system according to the fourth embodiment of the present disclosure.
Fig. 5 is a schematic view of an indoor event detecting system according to the fifth embodiment of the present disclosure.
Fig. 6 is a flow chart of an indoor event detecting method according to the sixth embodiment of the present disclosure.
Fig. 7A, Fig. 7B, Fig. 7C, Fig. 7D, Fig. 7E and Fig. 7F are schematic views of a door-closed from inside detection of the indoor event detecting method of the sixth embodiment.
Fig. 8A, Fig. 8B, Fig. 8C, Fig. 8D and Fig. 8E are schematic views of an occupancy detection of the indoor event detecting method of the sixth embodiment.
Fig. 9A, Fig. 9B, Fig. 9C, Fig. 9D, Fig. 9E and Fig. 9F are schematic views of a vital sign detection of the indoor event detecting method of the sixth embodiment.
Fig. 10A, Fig. 10B, Fig. 10C and Fig. 10D are schematic views of a water leakage detection of the indoor event detecting method of the sixth embodiment.

### DETAILED DESCRIPTION

Embodiments of the present disclosure will be described below with reference to the drawings. For the sake of clarity, many practical details will be explained together in the following statements. However, it should be understood that these practical details should not be used to limit the present disclosure. That is, these practical details are not necessary in embodiments of the present disclosure. In addition, for the sake of simplifying the drawings, some commonly used structures and components are shown in the drawings in a simple schematic manner; and repeated components may be represented by the same numbers.

In addition, the terms first, second, etc. are used herein to describe various elements or components, these elements or components should not be limited by these terms. Consequently, a first element or component discussed below could be termed a second element or component. Moreover, the combination of components in the present disclosure is not a combination that is generally known, conventional or customary in this field. The components themselves being or being not common knowledge cannot be used to determine whether the combination relationship can be easily completed by a person skilled in the technical field.

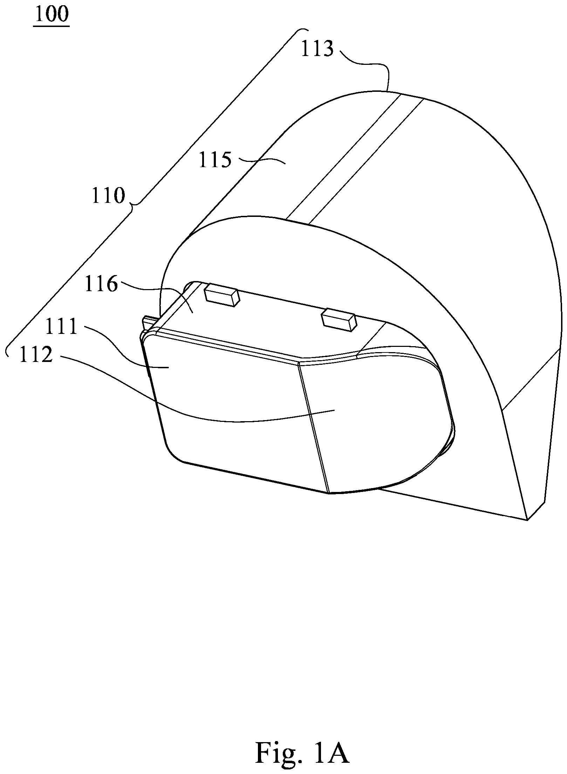

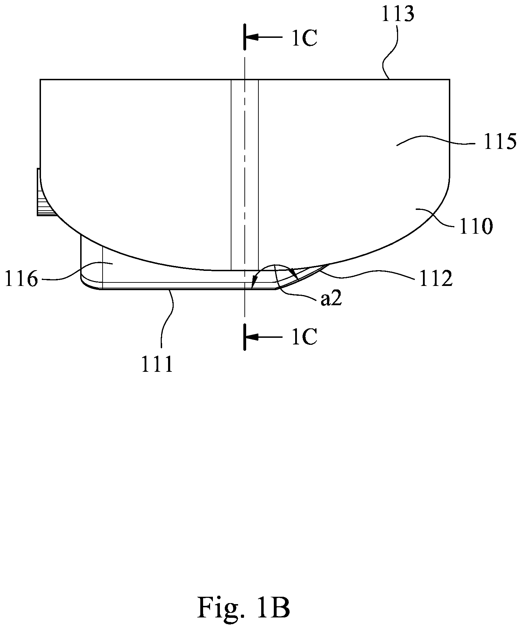

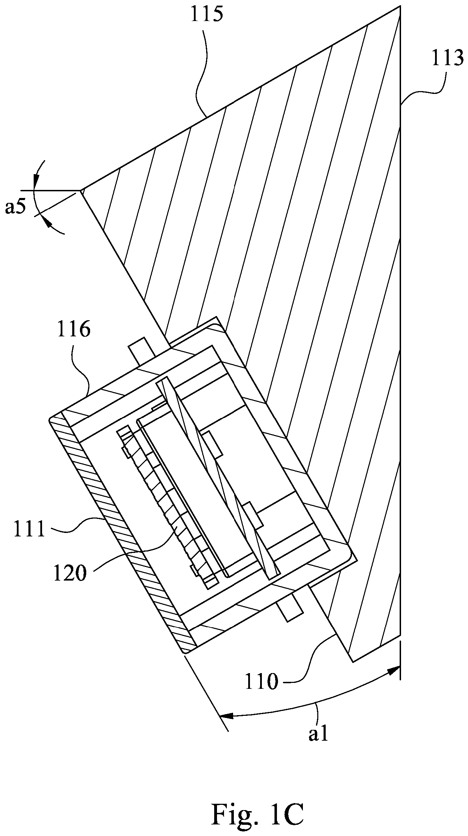

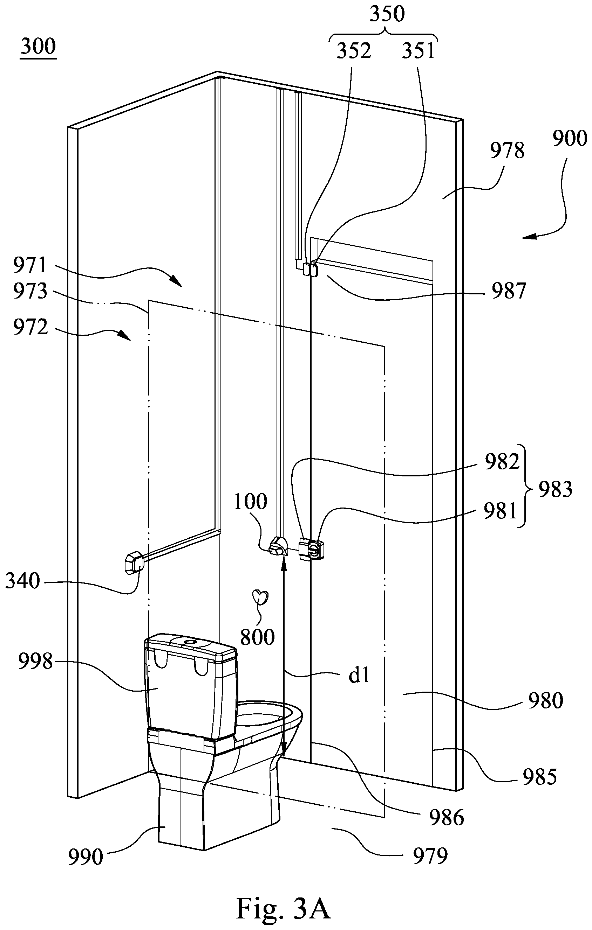

Fig. 1A is a three-dimensional view of an indoor radar device 100 according to the first embodiment of the present disclosure. Fig. 1B is a top view of the indoor radar device 100 in Fig. 1A. Fig. 1C is a cross-sectional view along line 1C-1C of the indoor radar device 100 in Fig. 1B. Fig. 3A is a schematic view of an indoor event detecting system 300 according to the third embodiment of the present disclosure. With reference to Fig. 1A to Fig. 1C and Fig. 3A, the indoor event detecting system 300 of the third embodiment is used to assist in describing the indoor radar device 100 of the first embodiment. The indoor radar device 100 includes a housing outer surface 110, which includes at least one mounting surface 113 and upper inclined surfaces 115, 116. The mounting surface 113 is configured to be connected to an indoor surface 978 of a room 900. The upper inclined surfaces 115, 116 are configured to inclinedly face a ceiling (not shown in drawings) of the room 900. An upper inclined angle a5 between each of the upper inclined surfaces 115, 116 and a floor (ground) 979 of the room 900 is between 15 degrees and 85 degrees, as shown in Fig. 1C. Therefore, each of the upper inclined surfaces 115, 116 is an inclined surface or a curved surface, which prevents the indoor radar device 100 from being used to place or hang items thereon, which interferes with radar signals, and the upper inclined angle a5 can increase drainage according to water droplet experiments. Specifically, the upper inclined angle a5 between the upper inclined surface 115 and the floor 979 of the room 900 in Fig. 1C is 30 degrees. An average surface roughness of the upper inclined surfaces 115, 116 may be less than 5 mm. A contact angle of the upper inclined surfaces 115, 116 may be greater than 30 degrees. The indoor surface 978 is a wall of the room 900.

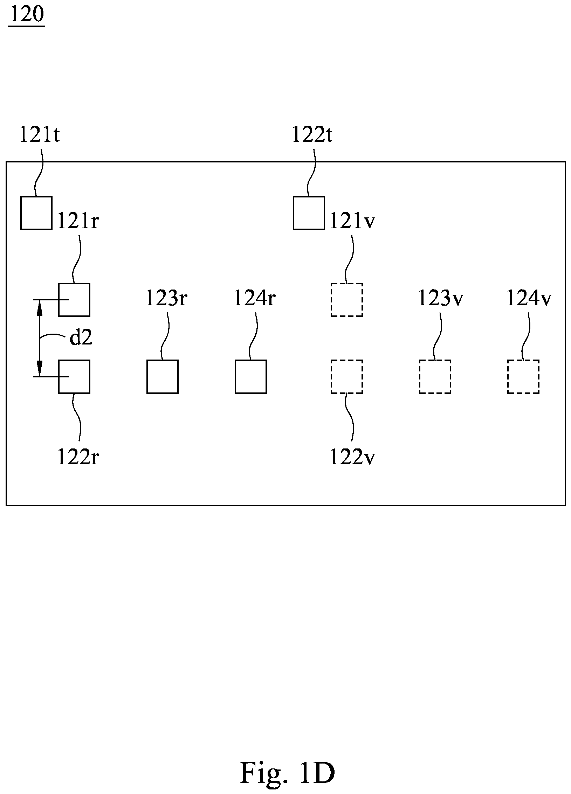

Fig. 1D is a schematic view of a first antenna array 120 of the indoor radar device 100 in Fig. 1C. With reference to Fig. 1C, Fig. 1D and Fig. 3A, in detail, the indoor radar device 100 may further include the first antenna array 120, which includes antennas 121r, 122r. The antennas 121r, 122r are arranged along a direction from the floor 979 to the ceiling, a distance (center-to-center distance) d2 between the antennas 121r, 122r is 0.5 times a wavelength, and the wavelength may be between 0.001 mm and 12 mm. Therefore, it helps to improve the resolution of the first antenna array 120 in the direction perpendicular to the floor 979. Specifically, the first antenna array 120 includes antennas 121t, 122t as transmitting antennas and antennas 121r, 122r, 123r, 124r, 121v, 122v, 123v, 124v as receiving antennas. The antennas 121t, 122t, 121r, 122r, 123r, 124r are real antennas, and the antennas 121v, 122v, 123v, 124v are virtual antennas. In addition, the equivalent virtual antenna effect can still be obtained after the locations of the transmitting antennas and the receiving antennas in the first antenna array according to the present disclosure are interchanged. That is, after the arrangement locations are interchanged, there are two receiving antennas, which are respectively located at the positions of the antennas 121t, 122t that are originally used as transmitting antennas, and there are four transmitting antennas, which are respectively located at the positions of the antennas 121r, 122r, 123r, 124r that are originally used as receiving antennas.

With reference to Fig. 1A, Fig. 1C and Fig. 3A, the housing outer surface 110 may further include a first emitting surface 111. The first antenna array 120 is adjacent to or disposed on the first emitting surface 111. The first emitting surface 111 is configured to be closer to the floor 979 than the upper inclined surfaces 115, 116 thereto, and the first emitting surface 111 is configured to inclinedly face the floor 979. A first angle a1 between the first emitting surface 111 and the mounting surface 113 may be between 5 degrees and 75 degrees, as shown in Fig. 1C. Therefore, it is helpful for the first emitting surface 111 to face the heart 800 or torso of the detected target (detected object) to obtain the vital signs of the detected target. Furthermore, the first angle a1 may be between 5 degrees and 40 degrees. Specifically, the first angle a1 in Fig. 1C is 30 degrees.

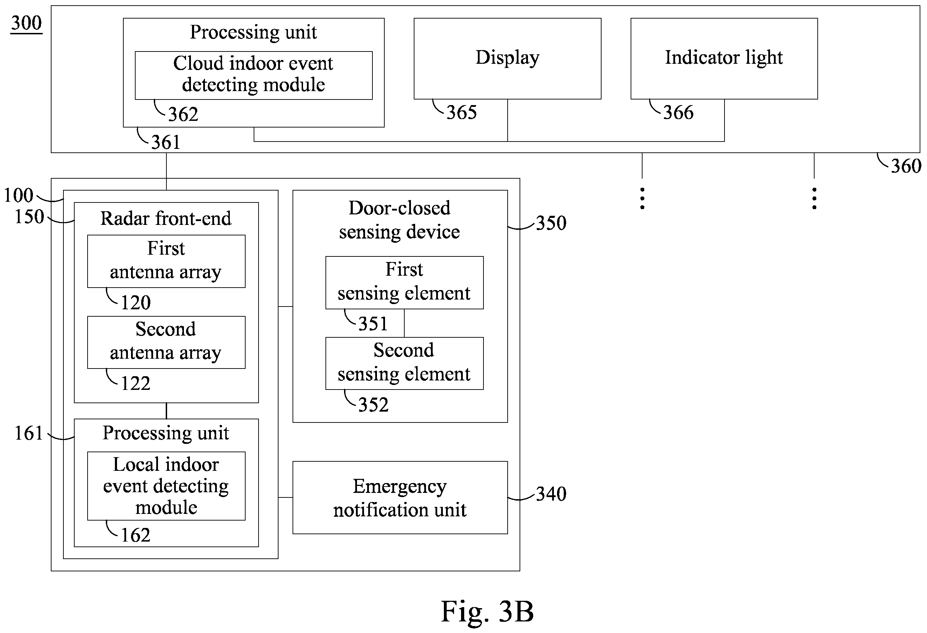

With reference to Fig. 1A and Fig. 1B, the indoor radar device 100 may further include a second antenna array 122 (shown in Fig. 3B). The housing outer surface 110 may further include a second emitting surface 112. The second antenna array 122 is adjacent to or disposed on the second emitting surface 112, and the second emitting surface 112 is configured to inclinedly face the floor 979. A second angle a2 between the first emitting surface 111 and the second emitting surface 112 may be between 105 degrees and 170 degrees, as shown in Fig. 1B. Therefore, it is beneficial to expand the detection range of the indoor radar device 100. Specifically, the second angle a2 in Fig. 1B is approximately 160 degrees.

Furthermore, the indoor radar device 100 may be configured for detecting whether the detected target satisfies a fall condition, and a distance d1 between the indoor radar device 100 and the floor 979 may be less than or equal to 90 cm. Therefore, the fall detection of the indoor radar device 100 is not required to consider the standing posture and is only required that the detected life signal returned close to the floor 979 belongs to the falling posture or the lying posture. Thus, the indoor radar device 100 does not need to use machine learning algorithms for the fall detection, so as to save costs and be suitable for various room sizes and configurations.

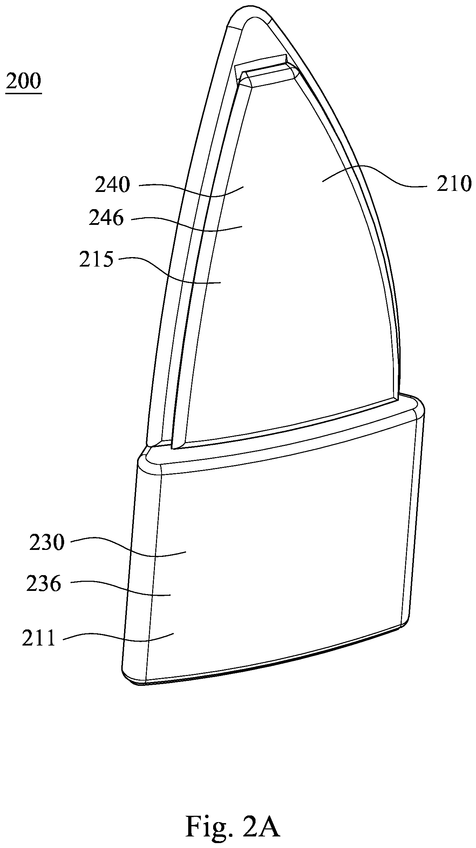

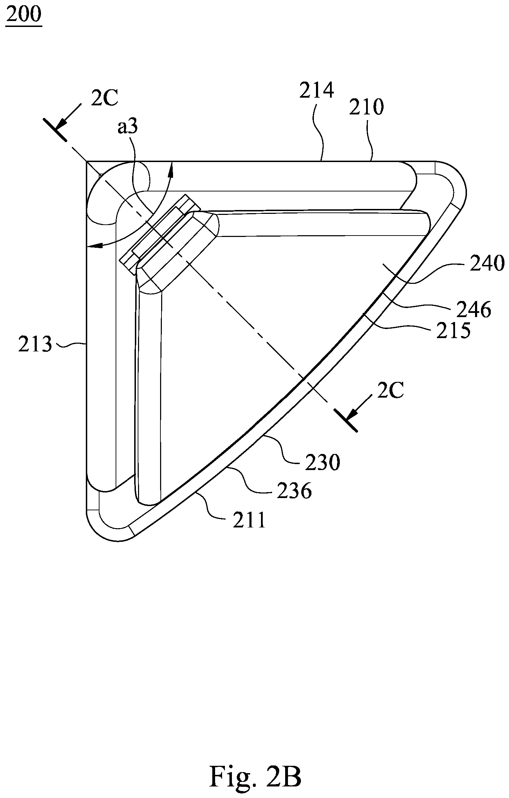

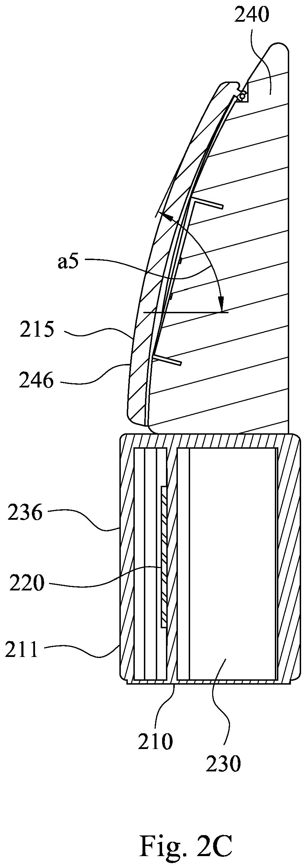

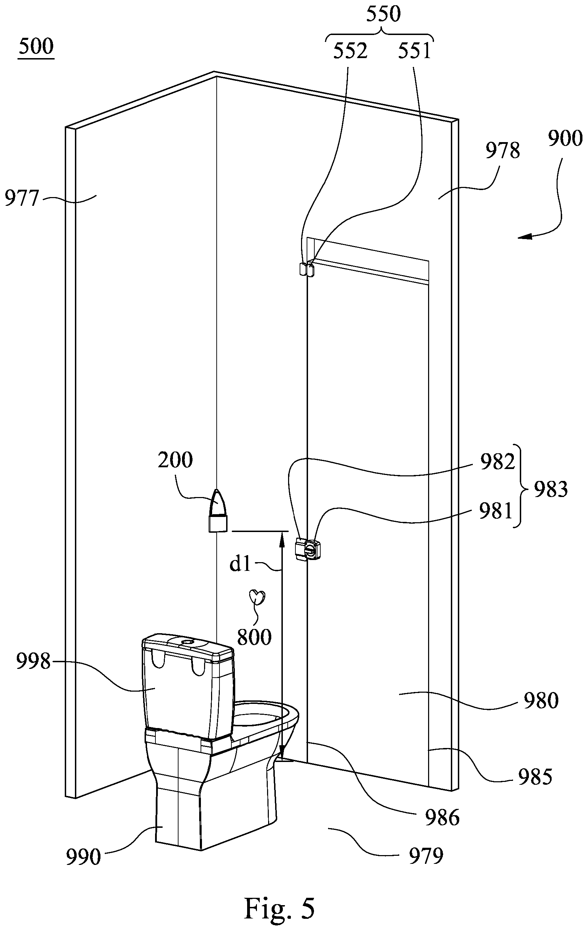

Fig. 2A is a three-dimensional view of an indoor radar device 200 according to the second embodiment of the present disclosure. Fig. 2B is a top view of the indoor radar device 200 in Fig. 2A. Fig. 2C is a cross-sectional view along line 2C-2C of the indoor radar device 200 in Fig. 2B. Fig. 5 is a schematic view of an indoor event detecting system 500 according to the fifth embodiment of the present disclosure. With reference to Fig. 2A to Fig. 2C and Fig. 5, the indoor event detecting system 500 of the fifth embodiment is used to assist in describing the indoor radar device 200 of the second embodiment. The indoor radar device 200 includes a housing outer surface 210, which includes mounting surfaces 213, 214 and an upper inclined surface 215. The mounting surfaces 213, 214 are configured to be respectively connected to indoor surfaces 977, 978 of a room 900. The upper inclined surface 215 is configured to inclinedly face a ceiling of the room 900. An upper inclined angle a5 between the upper inclined surface 215 and a floor 979 of the room 900 is between 15 degrees and 85 degrees, as shown in Fig. 2C.

Furthermore, the indoor radar device 200 is configured to be connected to the indoor surfaces 977, 978 of the room 900 and further includes a radar unit 230 and an emergency notification unit 240. The radar unit 230 includes a first antenna array 220, which includes at least two antennas (not shown in drawings). The two antennas are arranged along a direction from the floor 979 to the ceiling of the room 900. A distance (center-to-center distance) d2 between the two antennas is 0.5 times a wavelength, and the wavelength is between 0.001 mm and 12 mm. The emergency notification unit 240 is coupled (electrically and communicatively connected) to the radar unit 230. The housing outer surface 210 includes a radar outer surface 236 and an emergency notification outer surface 246. The radar outer surface 236 corresponds (is disposed correspondingly) to the radar unit 230, and the emergency notification outer surface 246 corresponds to the emergency notification unit 240 and is configured to be triggered by pressuring or contacting of a detected target in the room 900 to notify an emergency state. Therefore, by reducing the number of devices in the room 900, the popularity rate of the indoor radar device 200 is increased.

With reference to Fig. 2A, Fig. 2C and Fig. 5, in detail, the upper inclined surface 215 is a convex arc surface and has a plurality of normal directions. An average surface roughness of the upper inclined surface 215 is less than 5 mm. A contact angle of the upper inclined surface 215 is greater than 30 degrees. Each of the indoor surfaces 977, 978 is a wall surface of the room 900 or a surface in the room 900 being not parallel to the floor 979. Therefore, it is beneficial to avoid placing or hanging items that interfere with radar signals on the upper inclined surface 215, and let water droplets or dust to slide off not to be deposited on the housing outer surface 210 and the upper inclined surface 215 thereof, so as to avoid affecting nearby radiation field pattern.

With reference to Fig. 2A to Fig. 2C and Fig. 5, a number of the mounting surfaces 213, 214 is two, and a mounting surface angle a3 between the mounting surfaces 213, 214 may be between 70 degrees and 110 degrees. The upper inclined surface 215 is connected between the mounting surfaces 213, 214, and the upper inclined surface 215 is configured to be taped along a direction from the floor 979 to the ceiling. Therefore, the location of the indoor radar device 200 is beneficial to avoid affecting the activities of the detected target in the room 900. Specifically, the mounting surface angle a3 in Fig. 2B is 90 degrees.

The radar unit 230 includes a first antenna array 220, and the radar outer surface 236 includes a first emitting surface 211. The first antenna array 220 is adjacent to the first emitting surface 211, the first emitting surface 211 is configured to be closer to the floor 979 than the upper inclined surface 215 thereto, and the first emitting surface 211 is configured to be located at an extension direction from the upper inclined surface 215 to the floor 979 and perpendicular to the floor 979. Therefore, when the indoor radar device 200 needs to be installed at a slightly lower position, both the radar detection accuracy and the usability of the emergency notification unit 240 can be achieved.

The emergency notification unit 240 is configured to be physically connected to a part of the radar unit 230 away from the floor 979. The upper inclined surface 215 is located on the emergency notification outer surface 246. Therefore, the configuration design of the indoor radar device 200 is advantageous in improving the radar detection accuracy, and the detected person can easily access the emergency notification outer surface 246 when needed. Furthermore, the emergency notification unit 240 may include a movable component to provide the detected target or the user with fine-tuning of the installation height.

In addition, after adjusting (not shown in drawings), the first emitting surface 211 may be configured to inclinedly face the floor 979, and a first angle between the first emitting surface 211 and each of the mounting surfaces 213, 214 may be between 5 degrees and 75 degrees. Moreover, the mentioned first angle may be between 5 degrees and 40 degrees. Therefore, when the indoor radar device 200 needs to be installed at a slightly higher position, both the radar detection accuracy and the usability of the emergency notification unit 240 can be achieved.

With reference to Fig. 5, the indoor radar device 200 may be configured for detecting whether the detected target satisfies a fall condition, and a distance d1 between the indoor radar device 200 and the floor 979 is less than or equal to 90 cm.

Fig. 3B is a block diagram of the indoor event detecting system 300 in Fig. 3A. With reference to Fig. 1A, Fig. 3A and Fig. 3B, the indoor event detecting system 300 of the third embodiment according to the present disclosure includes the indoor radar device 100 of the first embodiment, a door-closed sensing device 350 and a control center 360. The indoor radar device 100 is configured to be disposed on the indoor surface 978 of the room 900 and includes the housing outer surface 110, which includes the upper inclined surfaces 115, 116. The door-closed sensing device 350 is configured to be disposed in the room 900 and may be wiredly or wirelessly communicatively connected to the indoor radar device 100. The control center 360 is wiredly or wirelessly communicatively connected to the indoor radar device 100 and the door-closed sensing device 350. The indoor event detecting system 300 is configured to: detect whether a door 980 of the room 900 starts to be in a door-closed state operated inside the room 900 (i.e., someone inside the room 900 activates the door 980 into the door-closed state) by the door-closed sensing device 350; perform an occupancy detection to determine whether there is a detected target in the room 900 and determine whether an overtime occupancy condition is satisfied by the indoor radar device 100 when it is detected that the door 980 starts to be in the door-closed state; perform a vital sign detection to determine whether a dangerous condition is satisfied by the indoor radar device 100 when it is detected that the door 980 starts to be in the door-closed state; and determine that an indoor dangerous event exists in the room 900 and generate an alarm when at least one of the overtime occupancy condition and the dangerous condition is satisfied. Therefore, the indoor event detecting system 300 is beneficial to promptly detect indoor dangerous events in the room 900 to avoid incidents.

In detail, the room 900 may be one of a toilet room, a bathroom and a dressing room, and is not limited thereto. Therefore, the indoor event detecting system 300 can be applied to a closed space that is not suitable for image detection due to factors such as taking off clothes and a single person is staying therein. In addition, the indoor event detecting system 300 can be used alone or in combination with an imaging system in a space suitable for image detection. Specifically, the room 900 in Fig. 3A is a toilet room, and the indoor event detecting system 300 of the third embodiment can be applied to a control center 360 and one or more rooms 900 (toilet rooms). As shown in Fig. 3A and Fig. 3B, the control center 360 may include a processing unit 361, a display 365 and an indicator light 366, and the processing unit 361 includes a cloud indoor event detecting module 362. The indoor radar device 100 includes a radar front-end 150 and a processing unit 161. The radar front-end 150 includes the first antenna array 120 and the second antenna array 122. The processing unit 161 includes a local indoor event detecting module 162. Both the cloud indoor event detecting module 362 and the local indoor event detecting module 162 are program codes.

The indoor event detecting system 300 may further include an emergency notification unit 340 configured to be disposed in the room 900. The control center 360 is wiredly or wirelessly communicatively connected to the indoor radar device 100, the door-closed sensing device 350 and the emergency notification unit 340. The wiredly communicative connection may refer to a communicative connection via a protocol of Ethernet, Internet of Things, CAN, UART, LIN or RS485, the wirelessly communicative connection may refer to a communicative connection via a protocol of WIFI, BLE, ASK/FSK or LoRa, and the present disclosure is not limited thereto. Furthermore, the control center 360 may be directly communicatively connected to the indoor radar device 100, the door-closed sensing device 350 and the emergency notification unit 340, respectively. Alternatively, the control center 360 may be directly communicatively connected to the indoor radar device 100, and the control center 360 is indirectly communicatively connected to the door-closed sensing device 350 and the emergency notification unit 340 via the indoor radar device 100.

The indoor surface 978 may be the wall surface of the room 900 or the surface in the room 900 being not parallel to the floor 979. The indoor radar device 100 further includes the first antenna array 120, and the housing outer surface 110 further includes the first emitting surface 111. The first antenna array 120 is adjacent to the first emitting surface 111, the first emitting surface 111 is configured to face the heart 800 of the detected target, and the distance d1 between the indoor radar device 100 and the floor 979 may be greater than 70 cm. Therefore, it is advantageous in improving the accuracy of the vital sign detection. Furthermore, the distance d1 between the indoor radar device 100 and the floor 979 may be greater than 70 cm and less than or equal to 90 cm, so that a single indoor radar device 100 can simultaneously achieve the purposes of the vital sign detection and the fall detection, thereby saving costs and reducing system complexity.

The door 980 includes a driving side 985 and an open side 986. The door-closed sensing device 350 may include a first sensing element 351 and a second sensing element 352. The first sensing element 351 is configured to be disposed on an upper end 987 of the open side 986, the second sensing element 352 is configured to be disposed on the indoor surface 978, and the first sensing element 351 and the second sensing element 352 are configured to detect a distance between each other. When the door 980 is in the door-closed state, the first sensing element 351 and the second sensing element 352 are disposed adjacent to each other. Therefore, the door-closed sensing device 350 is advantageous in not occupying the space of the room 900 and not hindering the movement of the detected target. Specifically, the door-closed sensing device 350 may be a Hall effect, infrared (IR) or microelectromechanical sensing device, the door 980 may be a pivoting door or a sliding door, and the present disclosure is not limited thereto.

A door lock assembly 983 of the room 900 includes a first lock element 981 and a second lock element 982. The first lock element 981 is disposed on the open side 986, and the second lock element 982 is disposed on the indoor surface 978.

In addition, after adjusting (not shown in drawings), the door-closed sensing device 350 may be configured to be disposed at at least one of the first lock element 981 and the second lock element 982 to detect whether the door 980 is in the door-closed state. For example, the first sensing element 351 may be configured to be disposed inside or on a surface of the first lock element 981, the second sensing element 352 may be configured to be disposed inside or on a surface of the second lock element 982, and the first sensing element 351 and the second sensing element 352 are configured to detect the distance between each other or the movement of the lock tongue. Therefore, integrating the door-closed sensing device 350 into the first lock element 981 and the second lock element 982 helps to improve the convenience of installing the indoor event detecting system 300.

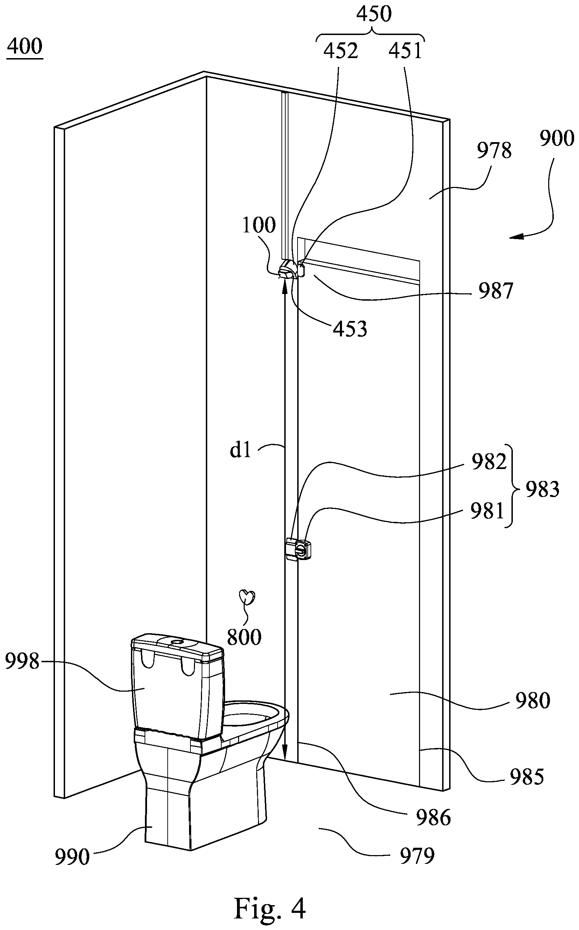

Fig. 4 is a schematic view of an indoor event detecting system 400 according to the fourth embodiment of the present disclosure. With reference to Fig. 1A and Fig. 4, the indoor event detecting system 400 of the fourth embodiment according to the present disclosure includes the indoor radar device 100 of the first embodiment, a door-closed sensing device 450 and a control center. The indoor radar device 100 is configured to be disposed on the indoor surface 978 of the room 900 and includes the housing outer surface 110, which includes the upper inclined surfaces 115, 116. The door-closed sensing device 450 is configured to be disposed in the room 900 and wiredly or wirelessly communicatively connected to the indoor radar device 100. The control center is wiredly or wirelessly communicatively connected to the indoor radar device 100 and the door-closed sensing device 450. The indoor event detecting system 400 is configured to: detect whether a door 980 of the room 900 starts to be in a door-closed state operated inside the room 900 by the door-closed sensing device 450; perform an occupancy detection to determine whether there is a detected target in the room 900 and determine whether an overtime occupancy condition is satisfied by the indoor radar device 100 when it is detected that the door 980 starts to be in the door-closed state; perform a vital sign detection to determine whether a dangerous condition is satisfied by the indoor radar device 100 when it is detected that the door 980 starts to be in the door-closed state; and determine that an indoor dangerous event exists in the room 900 and generate an alarm when at least one of the overtime occupancy condition and the dangerous condition is satisfied.

In detail, the room 900 in Fig. 4 is a toilet room. The first antenna array 120 of the indoor radar device 100 is adjacent to the first emitting surface 111, the first emitting surface 111 is configured to face the heart 800 of the detected target, and the distance d1 between the indoor radar device 100 and the floor 979 is greater than 70 cm.

The door-closed sensing device 450 includes a first sensing element 451 and a second sensing element 452. The first sensing element 451 is configured to be disposed on an upper end 987 of the open side 986, the second sensing element 452 is configured to be disposed on the indoor surface 978, and the first sensing element 451 and the second sensing element 452 are configured to detect a distance between each other. When the door 980 is in the door-closed state, the first sensing element 451 and the second sensing element 452 are disposed adjacent to each other.

The second sensing element 452 and the indoor radar device 100 are integrated into an apparatus 453. When the door 980 is in the door-closed state, the apparatus 453 is adjacent to the upper end 987 of the open side 986. Therefore, it helps to improve the convenience of installing the indoor event detecting system 400.

With reference to Fig. 2A and Fig. 5, the indoor event detecting system 500 of the fifth embodiment according to the present disclosure includes the indoor radar device 200 of the second embodiment, a door-closed sensing device 550 and a control center. The indoor radar device 200 is configured to be disposed on the indoor surface 978 of the room 900 and includes the housing outer surface 210, which includes the upper inclined surface 215. The door-closed sensing device 550 is configured to be disposed in the room 900 and wiredly or wirelessly communicatively connected to the indoor radar device 200. The control center is wiredly or wirelessly communicatively connected to the indoor radar device 200 and the door-closed sensing device 550. The indoor event detecting system 500 is configured to: detect whether a door 980 of the room 900 starts to be in a door-closed state operated inside the room 900 by the door-closed sensing device 450; perform an occupancy detection to determine whether there is a detected target in the room 900 and determine whether an overtime occupancy condition is satisfied by the indoor radar device 200 when it is detected that the door 980 starts to be in the door-closed state; perform a vital sign detection to determine whether a dangerous condition is satisfied by the indoor radar device 200 when it is detected that the door 980 starts to be in the door-closed state; and determine that an indoor dangerous event exists in the room 900 and generate an alarm when at least one of the overtime occupancy condition and the dangerous condition is satisfied.

In detail, the room 900 in Fig. 5 is a toilet room. The first antenna array 220 of the indoor radar device 200 is adjacent to the first emitting surface 211, the first emitting surface 211 is configured to face the heart 800 of the detected target, and the distance d1 between the indoor radar device 200 and the floor 979 is greater than 70 cm.

A number of the indoor surfaces 977, 978 is two. The indoor surfaces 977, 978 form a corner. The indoor radar device 200 is disposed on the corner and includes the radar unit 230 and the emergency notification unit 240, which is configured to be triggered by the detected target in the room 900 to notify an emergency state. The emergency notification unit 240 is physically connected to a part of the radar unit 230 away from the floor 979. Therefore, the installation position of the indoor radar device 200 can avoid affecting the activities of the detected target in the room 900 and provide installation convenience.

The door-closed sensing device 550 includes a first sensing element 551 and a second sensing element 552. The first sensing element 551 is configured to be disposed on an upper end 987 of the open side 986, the second sensing element 552 is configured to be disposed on the indoor surface 978, and the first sensing element 551 and the second sensing element 552 are configured to detect a distance between each other. When the door 980 is in the door-closed state, the first sensing element 551 and the second sensing element 552 are disposed adjacent to each other.

The indoor radar device 200 may be configured for detecting whether the detected target satisfies the fall condition, and the distance d1 between the indoor radar device 200 and the floor 979 is less than or equal to 90 cm. Moreover, the fall detection is not required to consider the standing posture and is only required that the detected life signal returned close to the floor 979 belongs to the falling posture or the lying posture, thereby not needing to use conventional fall recognition algorithms, such as convolutional neural networks (CNN) or recursive neural networks (RNN) to calculate characteristic values for classifying, so that it helps to save model training time and can be applied to various room sizes and configurations. Furthermore, for considering sitting and lying down postures, lives, such as cats and dogs, which usually move around on the ground, are excluded. Table 1 below shows the data corresponding to the first angle between the first emitting surface 211 and each of the mounting surfaces 213, 214 being 37.5 degrees (not shown in drawings) after the first emitting surface 211 of the indoor radar device 200 in the indoor event detecting system 500 is adjusted, while the detected target faces away from the indoor radar device 200, which is the most difficult to detect, is used as the harshest scenario. As shown in Table 1, cats and dogs can be identified through heart rate, and whether the detected target is a human can be accurately identified, so there is no need to classify and establish a parent database.

**Table 1**

| Positions of detected target | Detected target sitting on floor | | Detected target lying down | |
|---|---|---|---|---|
| | Human (heart rate) | Cat (heart rate) | Human (heart rate) | Cat (heart rate) |
| Front of toilet | 81 | 151 | 79 | 165 |
| Front right of toilet | 86 | 164 | 82 | 172 |
| Right of toilet | 82 | 167 | 89 | 158 |

In addition, the indoor event detecting system according to the present disclosure may include a control center and the configurations of the radar devices and door-closed sensing devices of the rooms 900 of at least two in the third to fifth embodiments.

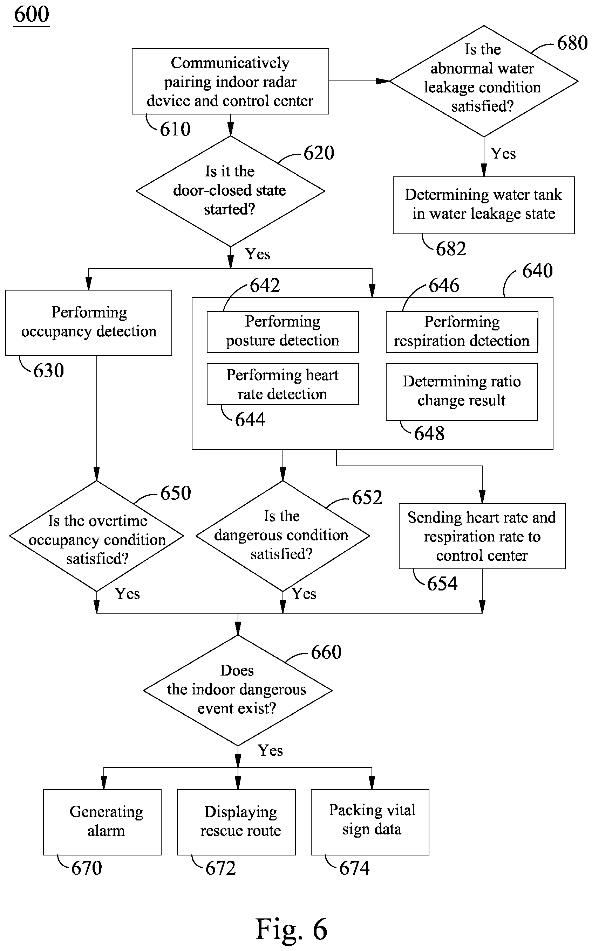

Fig. 6 is a flow chart of an indoor event detecting method 600 according to the sixth embodiment of the present disclosure. With reference to Fig. 1A, Fig. 3A and Fig. 6, the indoor event detecting method 600 includes steps 620, 630, 640, 650, 652, 660 and 670. The step 620 includes detecting whether the door 980 of the room 900 starts to be in the door-closed state operated inside the room 900 by the door-closed sensing device 350 disposed in the room 900. When (after) it is detected that the door 980 starts to be in the door-closed state in the step 620, the steps 630, 640 are performed; and when it is detected that the door 980 starts to change to a door-opened state, the steps 630, 640 may be stopped. The step 630 includes performing the occupancy detection. After the step 630, the step 650 is performed. The step 650 includes determining whether there is a detected target in the room 900 and determining whether the overtime occupancy condition is satisfied by the indoor radar device 100, and the indoor radar device 100 includes the housing outer surface 110, which includes the upper inclined surfaces 115, 116. The step 640 includes performing the vital sign detection. After the step 640, the step 652 is performed. The step 652 includes determining whether the dangerous condition is satisfied based on a vital sign detection result in the step 640 by the indoor radar device 100. After the steps 650, 652, the step 660 is performed. The step 660 includes determining whether the indoor dangerous event exists in the room 900 correspondingly based on whether at least one of the overtime occupancy condition and the dangerous condition is satisfied. When it is determined that the indoor dangerous event exists in the room 900, the step 670 is performed, and the step 670 includes generating the alarm. Therefore, the indoor event detecting method 600 can be used to promptly detect indoor dangerous events in the room 900 to avoid unfortunate events. In addition, the alarm can be presented in the control center 360, the room 900 or the rescuer's mobile device through at least one manner of an indicator light, a display image, a display text and a sound, and the present disclosure is not limited thereto.

In detail, the indoor event detecting method 600 may include a step 610. The step 610 includes communicatively pairing the indoor radar device 100 and the control center 360, and the indoor radar device 100 and the control center 360 are wirelessly connected via at least one of a frequency division multiplexing manner and a time division multiplexing manner. Therefore, it can prevent multiple indoor radar devices 100 in multiple rooms 900, respectively, from interfering with each other. Specifically, when there are multiple indoor radar devices 100 in the indoor event detecting system 300, the wireless communication unit of each of the indoor radar devices 100 has a specific identification code (ID), thereby communicating with the control center 360 to perform the aforementioned pairing, detecting loss of connection or multi-mode configuration. In addition, when it has been not detected that the door 980 starts to be in the door-closed state operated inside the room 900 in the step 620, the indoor radar device 100 can be in a standby polling or continuous detection mode.

With reference to Fig. 3A and Fig. 6, the room 900 may be a toilet room and divided into the door-closed detection space 971 and the occupancy detection space 972. The door-closed detection space 971 is adjacent to the door 980, the occupancy detection space 972 is adjacent to the toilet 990 in the room 900, and the boundary 973 shown in Fig. 3A separates the door-closed detection space 971 and the occupancy detection space 972. The step 620 of the indoor event detecting method 600 may include detecting whether the door 980 starts to be in the door-closed state operated inside the room 900 by the indoor radar device 100, which includes detecting whether the detected target moves from the door-closed detection space 971 to the occupancy detection space 972. Therefore, it helps to avoid false alarms caused by the detected target not being able to close or lock the door in time due to physical discomfort, or caused by the cleaning staff entering the room for cleaning. In addition, the detected target can be reminded by sound, indicator light or image that the door has not been closed or locked.

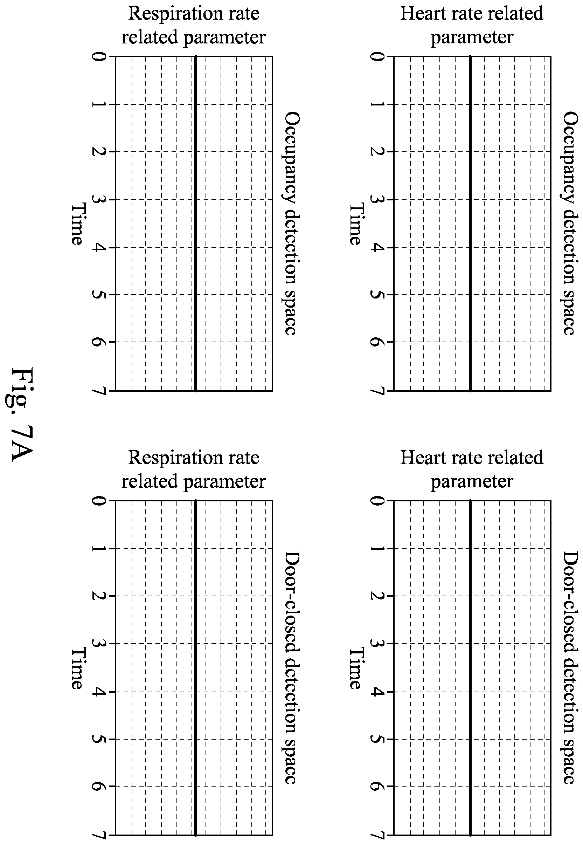

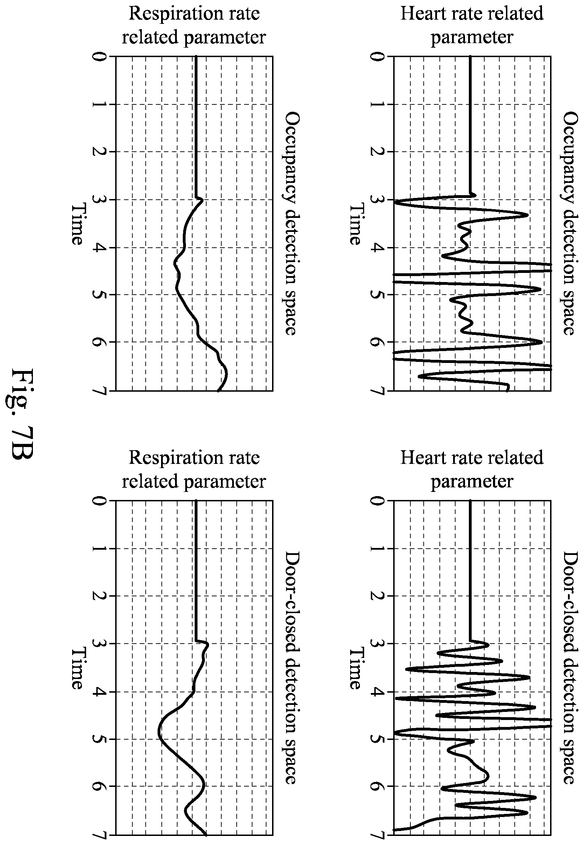

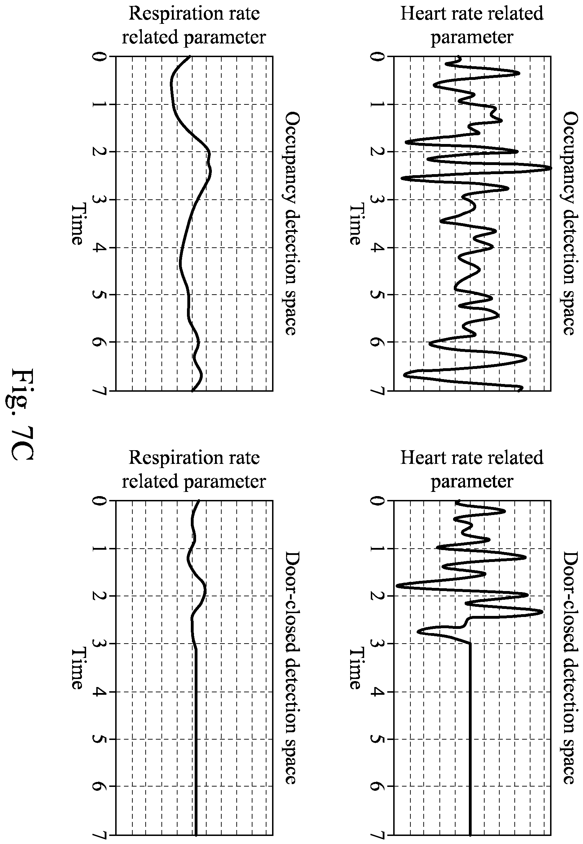

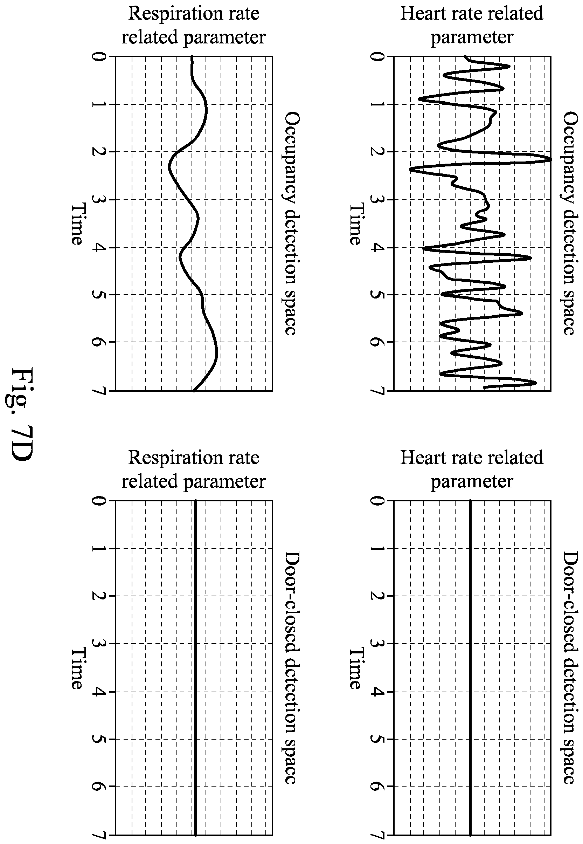

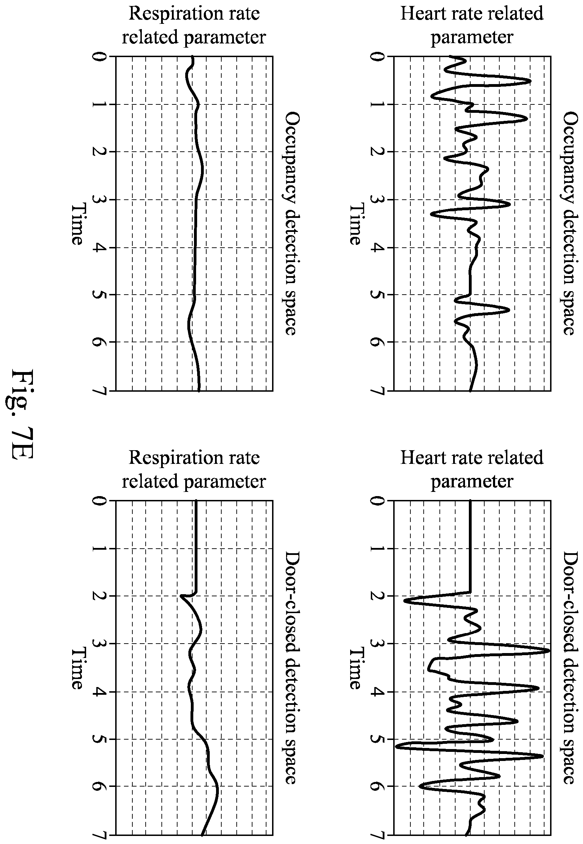

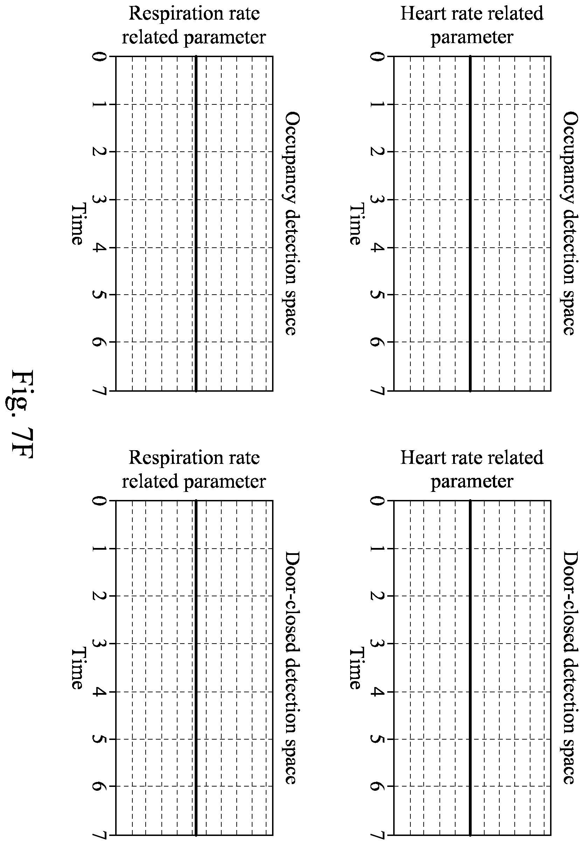

Fig. 7A, Fig. 7B, Fig. 7C, Fig. 7D, Fig. 7E and Fig. 7F are schematic views of the door-closed from inside detection (i.e., detecting whether someone inside the room 900 activates the door 980 into the door-closed state) in the step 620 of the indoor event detecting method 600 of the sixth embodiment, and Fig. 7A to Fig. 7F respectively show heart rate related parameters and respiration rate related parameters of the detected target detected in different action states in the door-closed detection space 971 and the occupancy detection space 972 in the room 900. In addition, the horizontal axis in each of Fig. 7A to Fig. 10D represents time, which may be in seconds or other units after processing, and the vertical axis therein represents a parameter, which may be the processed radar echo signal. With reference to Fig. 7A to Fig. 7F, Fig. 7A illustrates the detected parameters when the room 900 is empty or free of people and animals; Fig. 7B illustrates the detected parameters when the detected target opens the door 980 and then enters the room 900, and when the detected target enters, the door 980 has a large speed and angle change, the obvious signals of the door 980 are shown in the radar point cloud (not shown in drawings), and the signals related to the heart rate and the respiration rate in the door-closed detection space 971 and the occupancy detection space 972 begin to be detected; Fig. 7C illustrates the detected parameters during the process of the detected target moving from the door-closed detection space 971 to the occupancy detection space 972; Fig. 7D illustrates the detected parameters during the process of the detected target using the toilet 990 in the occupancy detection space 972, and the signals of the door-closed detection space 971 no longer exist when the detected target using the toilet 990 in the occupancy detection space 972; Fig. 7E illustrates the detected parameters during the process that the detected target ends using the toilet 990 in the occupancy detection space 972, stands up, and then moves to the door-closed detection space 971; and Fig. 7F illustrates the detected parameters after the detected target leaving the room 900.

With reference to Fig. 6, the overtime occupancy condition in the step 650 may include that an occupancy time value of the detected target in the room 900 is greater than an occupancy time threshold, and the occupancy time threshold is a threshold value between 3 minutes and 90 minutes. Therefore, it can meet the needs of the golden intervention time of long-term deep respiration within 90 minutes and the golden intervention time of heart rate combined with abnormal respiration within 5 minutes. Furthermore, a number of the occupancy time threshold may be multiple, and a larger occupancy time threshold corresponds to a stronger alarm.

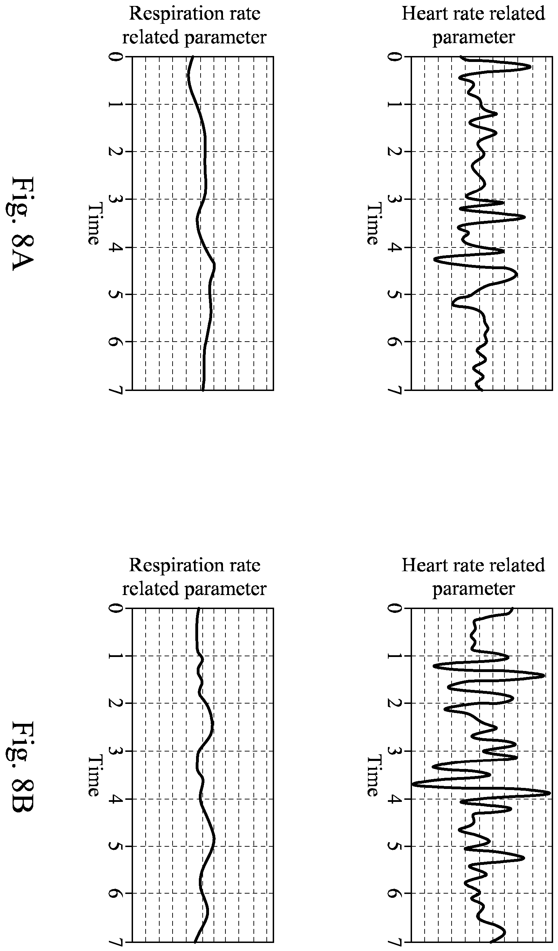

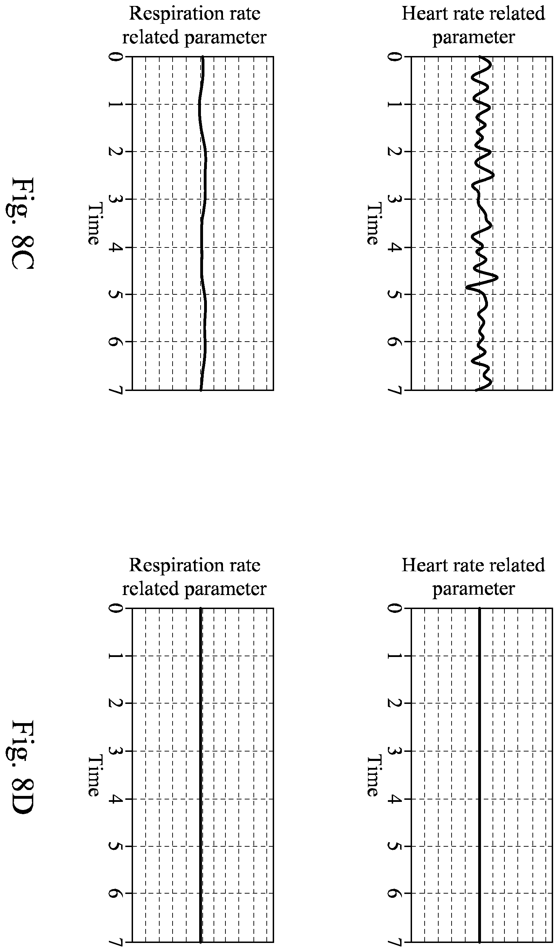

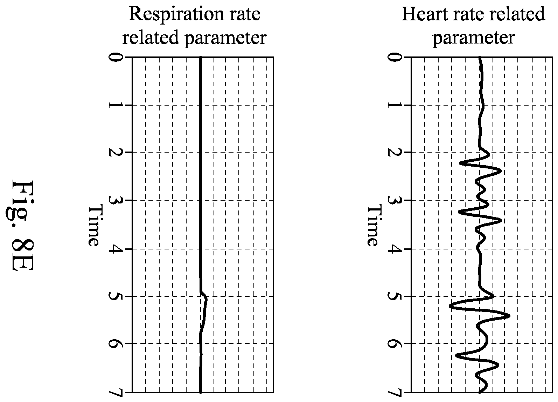

Fig. 8A, Fig. 8B, Fig. 8C, Fig. 8D and Fig. 8E are schematic views of the occupancy detection in the step 650 of the indoor event detecting method 600 of the sixth embodiment, and Fig. 8A to Fig. 7E respectively show heart rate related parameters and respiration rate related parameters of the detected target detected in different action states in the room 900. With reference to Fig. 8A to Fig. 8E, Fig. 8A illustrates the detected parameters when the detected target in the room 900 uses the toilet 990 and does not use a smartphone at the same time; Fig. 8B illustrates the detected parameters when the detected target in the room 900 uses the toilet 990 and uses a smartphone at the same time, and when the detected target uses the toilet 990, it can be clearly detected that there is a life occupying the room 900, and the indoor event detecting method 600 according to the present disclosure can filter the tiny movements of the detected target to avoid small movements of operating the smartphone from being merged into heart rate signals to misjudge the events; Fig. 8C illustrates the detected parameters when the detected target in the room 900 feels unwell, and compared with Fig. 8A and Fig. 8B, it can be seen with the naked eye that Fig. 8C is significantly different from Fig. 8A and Fig. 8B, so it can be used for the step 660 to determine whether there is an indoor dangerous event in the room 900; Fig. 8D illustrates the detected parameters when the toilet paper in the room 900 shakes slightly; and Fig. 8E illustrates the detected parameters when the toilet paper in the room 900 shakes severely, and compared with Fig. 8A and Fig. 8B, the indoor event detecting method 600 according to the present disclosure is able to identify environmental disturbances of non-biological signals under the status without closing door signals.

Performing the vital sign detection of the step 640 may include performing at least one of steps 642, 644, 646. The step 642 includes performing a posture detection, which includes detecting whether a detected target stationary condition is satisfied and whether a fall condition is satisfied based on at least five sampling points by the indoor radar device 100, which is a posture detection result. The step 644 includes performing a heart rate detection, which includes detecting a heart rate related to a time to determine whether an abnormal heart rate condition is satisfied, which is a heart rate detection result. The step 646 includes performing a respiration detection, which includes detecting a respiration rate related to the time to determine whether an abnormal respiration condition is satisfied, which is a respiration detection result. The indoor event detecting method 600 may further include a step 654, which includes sending (transmitting) the heart rate and the respiration rate to the control center 360 by the indoor radar device 100 and storing the heart rate and the respiration rate in the control center 360. The step 652 includes determining whether the dangerous condition is satisfied based on corresponding at least one of the posture detection result, the heart rate detection result and the respiration detection result in the steps 642, 644, 646, respectively. The step 660 includes determining whether the indoor dangerous event exists in the room 900 based on whether at least one of the overtime occupancy condition and the dangerous condition is satisfied. Therefore, for example, in a normal quiet state, the normal respiration rate of an adult is usually between 12 times/minute and 20 times/minute, and the steady-state baseline of the current detected target can be calculated in the early stage. Changes in accelerated breathing and shallow breathing may be manifestations of anxiety. Prolonged deep breathing may be manifestations of chronic obstructive pulmonary disease (COPD), COVID-19, or asthma. The abnormal respiration conditions in the step 646 of the indoor event detecting method 600 may include accelerated breathing, shallow breathing, prolonged deep breathing, slowing down or stopping of breathing. In addition, when the heart rate and/or the respiration is abnormal, the distance d2 between the antennas 121r, 122r of the first antenna array 120 being 0.5 times the wavelength and at least five sampling points captured for the detected target are conducive to accurately detect whether a fall exist in the step 642.

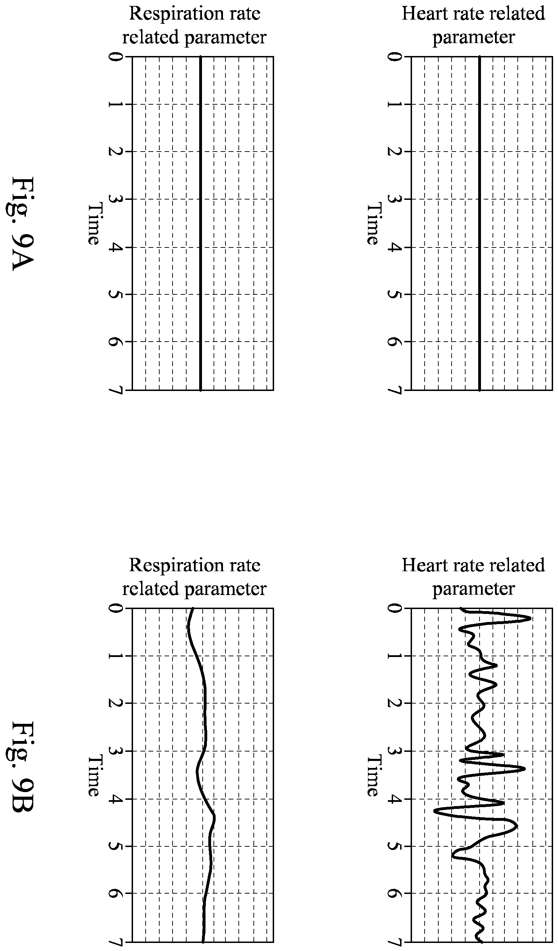

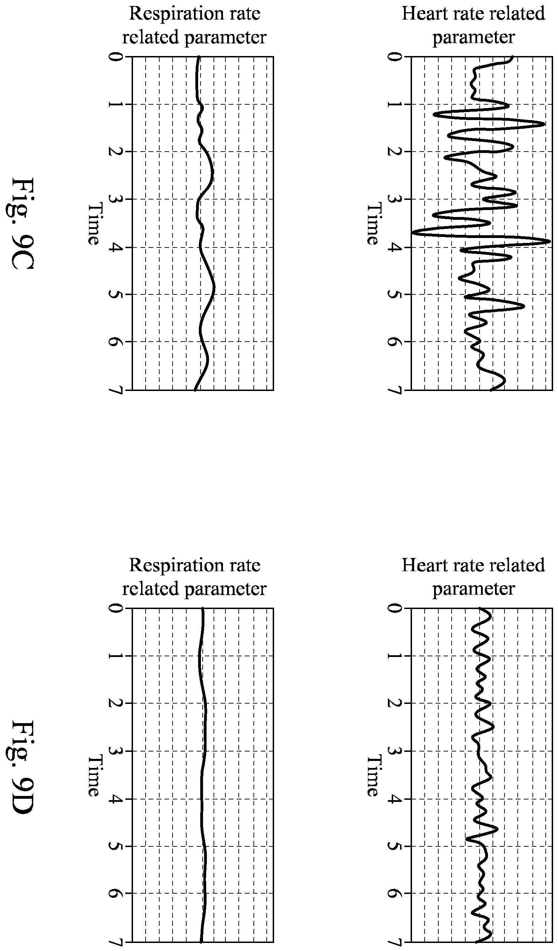

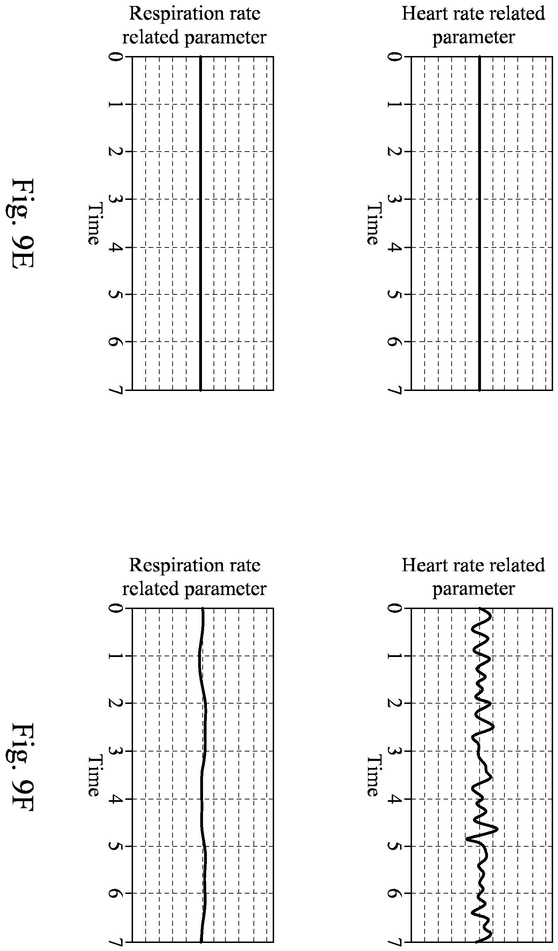

Fig. 9A, Fig. 9B, Fig. 9C, Fig. 9D, Fig. 9E and Fig. 9F are schematic views of the vital sign detection in the step 640 (specifically, the heart rate detection and the respiration detection in the steps 644, 646) of the indoor event detecting method 600 of the sixth embodiment, and Fig. 9A to Fig. 9F respectively show heart rate related parameters and respiration rate related parameters of the detected target detected in different action states in the room 900. With reference to Fig. 9A to Fig. 9F, Fig. 9A illustrates the detected parameters when the room 900 is empty; Fig. 9B illustrates the detected parameters when the detected target in the room 900 uses the toilet 990 while not being distracted by other matters and has stable vital signs and no danger suspicion; Fig. 9C illustrates the detected parameters when the detected target in the room 900 uses the toilet 990 and uses a smartphone at the same time, the vital signs thereof are stable, and there is no danger suspicion, and the detected target has movement phenomenon due to the use of the smartphone, etc., and the heartbeat and breathing changes greatly; Fig. 9D illustrates the detected parameters when the heartbeat and breathing of the detected target in room 900 slow down, based on this, it is inferred that the detected target may have heart failure, difficulty breathing or even life-threatening conditions, and the alarm is generated in the step 670; Fig. 9E illustrates the detected parameters when the heart rate and respiration rate related parameters of the detected target in the room 900 cannot be detected (it has been determined in the step 650 that there is a detected target in the room 900), based on this, it is inferred that the detected target may in a state of heart failure, difficulty breathing, shock, apnea or death, and the alarm is generated in the step 670; and Fig. 9F illustrates the detected parameters when the heartbeat and breathing of the detected target in room 900 slow down, based on this, it is inferred that the detected target may have heart failure, difficulty breathing or even life-threatening conditions, and at this time, the detected target triggers the emergency notification unit 340 and the alarm is generated in the step 670.

Performing the vital sign detection of the step 640 may include performing at least one of steps 642, 644, 646, 648. The step 648 includes determining whether a ratio change value is greater than a ratio change threshold, which is a ratio change result, and the ratio change value is a change value varied with the time of a ratio of the heart rate to the respiration rate. The step 652 includes determining whether the dangerous condition is satisfied based on corresponding at least one of the posture detection result, the heart rate detection result, the respiration detection result and the ratio change result. The step 660 includes determining whether the indoor dangerous event exists in the room 900 based on whether at least one of the overtime occupancy condition and the dangerous condition is satisfied. Therefore, in a normal quiet state, the heart rate is between 60 beats/minute and 100 beats/minute, and the respiration rate is between 12 times/minute and 20 times/minute, so the ratio of the heart rate to the respiration rate usually shows a certain (nearly fixed) ratio. Sudden abnormal changes in the ratio of the heart rate to the respiration rate may indicate potential problems, such as heart failure, which is characterized by shortness of breath and rapid heartbeat, or myocardial infarction, which is characterized by rapid breathing and irregular heartbeat. The ratio change threshold in the step 648 of the indoor event detecting method 600 helps to detect the aforementioned situations in which rescue intervention may be required.

The indoor dangerous event may correspond one of a plurality of danger levels. The danger levels include an intervention level and an aggravation level. The aggravation level may be that the life signal of the detected target in the room 900 disappears or the emergency notification unit 340 is triggered. The indicator light 366 of the control center 360 is configured to correspondingly show a plurality of indication states respectively based on the danger levels. The indicator light 366 can be an LED indicator light. A first color can be displayed correspondingly to the intervention level, and a second color can be displayed correspondingly to the aggravation level. The alarm in the step 670 includes showing one of the indication states by the indicator light 366. The indoor event detecting method 600 may further include steps 672, 674. When it is determined that the indoor dangerous event exists in the room 900, the steps 672, 674 may further be performed. The step 672 includes displaying a rescue route, which includes a route from a first aid equipment to the room 900, by at least one of the display 365 of the control center 360 and a mobile device of a rescuer. The step 674 includes packing the heart rate and the respiration rate being stored into a vital sign data by the control center 360 for the rescuer to obtain from the at least one of the control center 360 and the mobile device, so as to assist medical staff in making rescue decisions. Therefore, the first aid equipment can include a first aid kit and an AED (automated external defibrillators). When the control center 360 introduces the location positioning of the first aid equipment, the rescue route can be planned based on the location of the nearest first aid equipment.

The indoor event detecting method 600 may further include steps 680, 682. The step 680 includes detecting whether a water tank 998 in the room 900 satisfies an abnormal water leakage condition by the indoor radar device 100, and the room 900 is the toilet room. In the step 680, the indoor radar device 100 may detect once every time interval (e.g., 30 minutes). When the water leakage condition is satisfied in the step 680, the step 682 is performed. The step 682 includes determining that the water tank 998 is in a water leakage state, and notifying the control center 360 may be further performed. Therefore, the indoor event detecting method 600 is advantageous in providing broader event detection for the room 900.

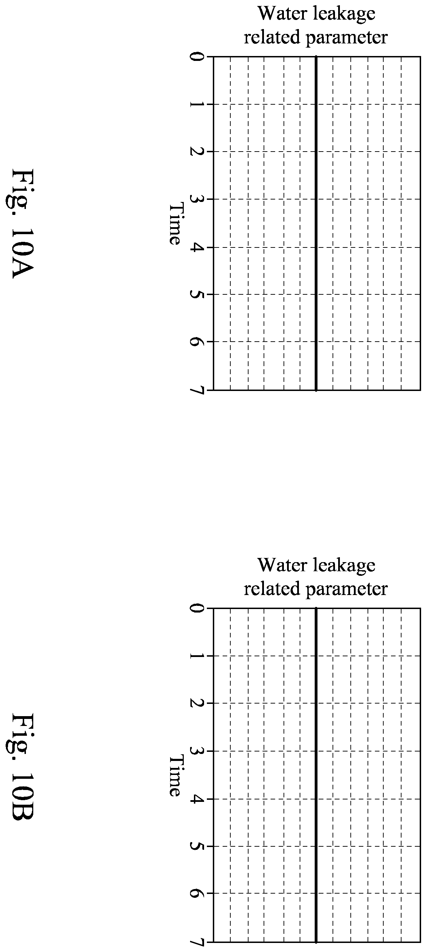

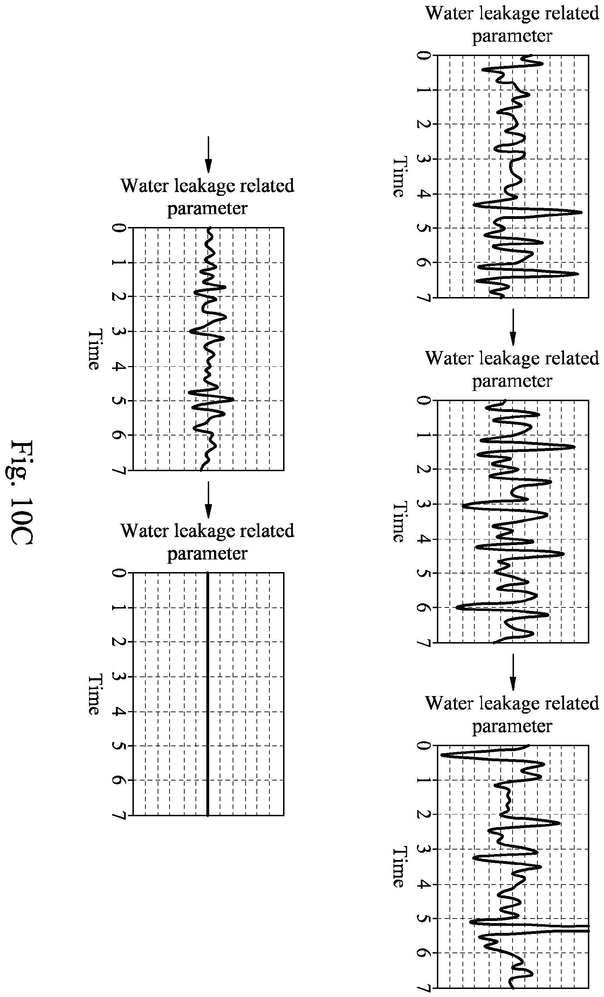

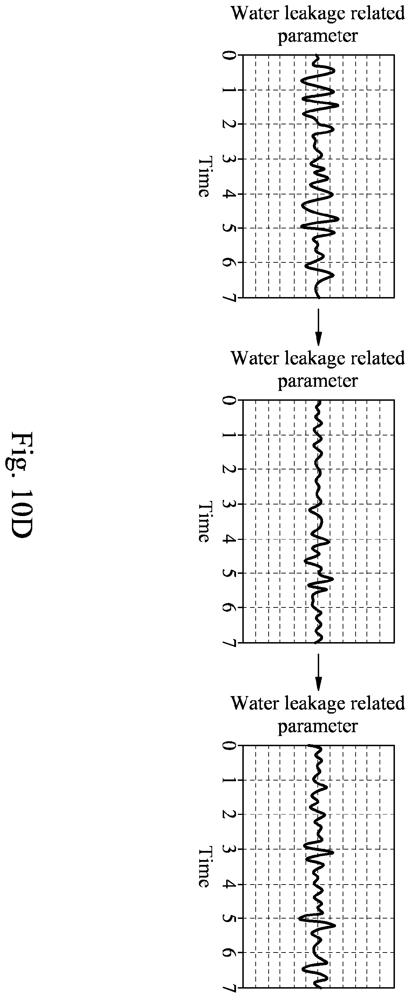

Fig. 10A, Fig. 10B, Fig. 10C and Fig. 10D are schematic views of the water leakage detection of the steps 680, 682 of the indoor event detecting method 600 of the sixth embodiment, and Fig. 10A to Fig. 10D respectively show water leakage (drip) related parameters detected for the water tank 998 of the room 900 in different states With reference to Fig. 10A to Fig. 10D, Fig. 10A illustrates the detected parameters when the water tank 998 is stationary and does not leak, Fig. 10B illustrates the detected parameters during the closing process of the lid (not shown in drawings) of the toilet 990, Fig. 10C illustrates the detected parameters during the flushing process of the toilet 990, and Fig. 10D illustrates the detected parameters during the water leakage or replenishment process of the water tank 998.

## Claims

1. An indoor radar device (100), **characterized in** comprising:
a housing outer surface (110) comprising at least one mounting surface (113) and an upper inclined surface (115), wherein the mounting surface (113) is configured to be connected to an indoor surface (978) of a room (900), the upper inclined surface (115) is configured to inclinedly face a ceiling of the room (900), and an upper inclined angle (a5) between the upper inclined surface (115) and a floor (979) of the room (900) is between 15 degrees and 85 degrees.

2. The indoor radar device (100) of claim 1, wherein the upper inclined surface (115) is a convex arc surface and has a plurality of normal directions, an average surface roughness of the upper inclined surface (115) is less than 5 mm, a contact angle of the upper inclined surface (115) is greater than 30 degrees, and the indoor surface (978) is a wall surface of the room (900) or a surface in the room (900) being not parallel to the floor (979).

3. The indoor radar device (100) of any one of claims 1-2, further comprising:
a first antenna array (120) comprising at least two antennas (121r, 122r), wherein the two antennas (121r, 122r) are arranged along a direction from the floor (979) to the ceiling, a distance (d2) between the two antennas (121r, 122r) is 0.5 times a wavelength, and the wavelength is between 0.001 mm and 12 mm.

4. The indoor radar device (100) of any one of claims 1-3, wherein the housing outer surface (110) further comprises a first emitting surface (111), the first antenna array (120) is adjacent to or disposed on the first emitting surface (111), the first emitting surface (111) is configured to be closer to the floor (979) than the upper inclined surface (115) thereto, the first emitting surface (111) is configured to inclinedly face the floor (979), and a first angle (a1) between the first emitting surface (111) and the mounting surface (113) is between 5 degrees and 75 degrees.

5. The indoor radar device (100) of any one of claims 1-4, further comprising:
a second antenna array (122);
wherein the housing outer surface (110) further comprises a second emitting surface (112), the second antenna array (122) is adjacent to or disposed on the second emitting surface (112), the second emitting surface (112) is configured to inclinedly face the floor (979), and a second angle (a2) between the first emitting surface (111) and the second emitting surface (112) is between 105 degrees and 170 degrees.

6. The indoor radar device (200) of any one of claims 1-5, wherein a number of the at least one mounting surface (213, 214) is two, a mounting surface angle (a3) between the two mounting surfaces (213, 214) is between 70 degrees and 110 degrees, the upper inclined surface (215) is connected between the two mounting surfaces (213, 214), and the upper inclined surface (215) is configured to be taped along a direction from the floor (979) to the ceiling.

7. The indoor radar device (200) of any one of claims 1-6, further comprising:
a radar unit (230); and
an emergency notification unit (240) configured to be triggered by a detected target in the room (900) to notify an emergency state, wherein the emergency notification unit (240) is configured to be connected to a part of the radar unit (230) away from the floor (979);
wherein a radar outer surface (236) of the housing outer surface (210) corresponds to the radar unit (230), an emergency notification outer surface (246) of the housing outer surface (210) corresponds to the emergency notification unit (240), and the upper inclined surface (215) is located on the emergency notification outer surface (246).

8. The indoor radar device (200) of any one of claims 1-7, wherein the radar unit (230) comprises a first antenna array (220), the radar outer surface (236) comprises a first emitting surface (211), the first antenna array (220) is adjacent to or disposed on the first emitting surface (211), and the first emitting surface (211) is configured to be located at an extension direction from the upper inclined surface (215) to the floor (979).

9. An indoor radar device (200), **characterized in** being configured to be connected to an indoor surface (978) of a room (900) and comprising:
a radar unit (230) comprising a first antenna array (220), which comprises at least two antennas, wherein the two antennas are arranged along a direction from a floor (979) to a ceiling of the room (900), a distance (d2) between the two antennas is 0.5 times a wavelength, and the wavelength is between 0.001 mm and 12 mm;
an emergency notification unit (240) coupled to the radar unit (230); and
a housing outer surface (210) comprising a radar outer surface (236) and an emergency notification outer surface (246), wherein the radar outer surface (236) corresponds to the radar unit (230), and the emergency notification outer surface (246) corresponds to the emergency notification unit (240) and is configured to be triggered by a detected target in the room (900) to notify an emergency state.

10. The indoor radar device (200) of claim 9, wherein the emergency notification unit (240) is configured to be connected to a part of the radar unit (230) away from the floor (979).

11. The indoor radar device (200) of any one of claims 9-10, wherein the radar outer surface (236) comprises a first emitting surface (211), the first antenna array (220) is adjacent to or disposed on the first emitting surface (211), and the first emitting surface (211) is perpendicular to the floor (979).

12. The indoor radar device (200) of any one of claims 9-11, wherein the emergency notification outer surface (246) comprises an upper inclined surface (215), which is configured to inclinedly face a ceiling of the room (900), and the first emitting surface (211) is configured to be closer to the floor (979) than the upper inclined surface (215) thereto.

13. The indoor radar device (200) of any one of claims 9-12, wherein the housing outer surface (210) further comprises two mounting surfaces (213, 214), which are configured to be connected to the indoor surface (978), and a mounting surface angle (a3) between the two mounting surfaces (213, 214) is between 70 degrees and 110 degrees.

14. The indoor radar device (200) of any one of claims 9-13, wherein the housing outer surface (210) further comprises at least one mounting surface (213), which is configured to be connected to the indoor surface (978), the housing outer surface (210) comprises a first emitting surface (211), the first antenna array (220) is adjacent to or disposed on the first emitting surface (211), the first emitting surface (211) is configured to inclinedly face the floor (979), and a first angle (a1) between the first emitting surface (211) and the mounting surface (213) is between 5 degrees and 75 degrees.

15. The indoor radar device (200) of any one of claims 9-14, wherein the indoor radar device (200) is configured for detecting whether the detected target satisfies a fall condition, and a distance (d1) between the indoor radar device (200) and the floor (979) is less than or equal to 90 cm.

16. An indoor event detecting system (300), **characterized in** comprising:
at least one indoor radar device (100) configured to be disposed on at least one indoor surface (978) of at least one room (900) and comprising at least one housing outer surface (110), which comprises an upper inclined surface (115);
at least one door-closed sensing device (350) configured to be disposed in the at least one room (900); and
a control center (360) wiredly or wirelessly communicatively connected to the at least one indoor radar device (100) and the at least one door-closed sensing device (350);
wherein the indoor event detecting system (300) is configured to:
detect whether a door (980) of the room (900) starts to be in a door-closed state operated inside the room (900) by the door-closed sensing device (350);
perform an occupancy detection to determine whether there is a detected target in the room (900) and determine whether an overtime occupancy condition is satisfied by the indoor radar device (100) when it is detected that the door (980) starts to be in the door-closed state;
perform a vital sign detection to determine whether a dangerous condition is satisfied by the indoor radar device (100) when it is detected that the door (980) starts to be in the door-closed state; and
determine that an indoor dangerous event exists in the room (900) and generate an alarm when at least one of the overtime occupancy condition and the dangerous condition is satisfied.

17. The indoor event detecting system (300) of claim 16, wherein the room (900) is one of a toilet room, a bathroom and a dressing room.

18. The indoor event detecting system (300) of any one of claims 16-17, wherein the indoor surface (978) is a wall surface of the room (900) or a surface in the room (900) being not parallel to a floor (979), the indoor radar device (100) further comprises a first antenna array (120), the housing outer surface (110) further comprises a first emitting surface (111), the first antenna array (120) is adjacent to or disposed on the first emitting surface (111), the first emitting surface (111) is configured to face a heart (800) of the detected target, and a distance (d1) between the indoor radar device (100) and the floor (979) is greater than 70 cm.

19. The indoor event detecting system (300) of any one of claims 16-18, wherein the door (980) comprises a driving side (985) and an open side (986), the door-closed sensing device (350) comprises a first sensing element (351) and a second sensing element (352), the first sensing element (351) is configured to be disposed on an upper end (987) of the open side (986), the second sensing element (352) is configured to be disposed on the indoor surface (978), and the first sensing element (351) and the second sensing element (352) are configured to detect a distance between each other;
wherein when the door (980) is in the door-closed state, the first sensing element (351) and the second sensing element (352) are disposed adjacent to each other.

20. The indoor event detecting system (500) of any one of claims 16-19, wherein a number of the at least one indoor surface (977, 978) is two, the two indoor surfaces (977, 978) form a corner, the indoor radar device (200) is disposed on the corner and further comprises a radar unit (230) and an emergency notification unit (240), which is configured to be triggered by a detected target in the room (900) to notify an emergency state, and the emergency notification unit (240) is configured to be connected to a part of the radar unit (230) away from a floor (979).

21. The indoor event detecting system (300) of any one of claims 16-20, wherein the door (980) comprises a driving side (985) and an open side (986), a door lock assembly (983) of the room (900) comprises a first lock element (981) and a second lock element (982), the first lock element (981) is disposed on the open side (986), and the second lock element (982) is disposed on the indoor surface (978);
wherein the door-closed sensing device (350) is configured to be disposed at at least one of the first lock element (981) and the second lock element (982) to detect whether the door (980) is in the door-closed state.

22. An indoor event detecting method (600), **characterized in** comprising:
detecting whether a door (980) of a room (900) starts to be in a door-closed state operated inside the room (900) by a door-closed sensing device (350) disposed in the room (900);
performing an occupancy detection to determine whether there is a detected target in the room (900) and determine whether an overtime occupancy condition is satisfied by an indoor radar device (100) when it is detected that the door (980) starts to be in the door-closed state, wherein the indoor radar device (100) comprises a housing outer surface (110), which comprises an upper inclined surface (115);
performing a vital sign detection to determine whether a dangerous condition is satisfied by the indoor radar device (100) when it is detected that the door (980) starts to be in the door-closed state; and
determining that an indoor dangerous event exists in the room (900) and generating an alarm when at least one of the overtime occupancy condition and the dangerous condition is satisfied.

23. The indoor event detecting method (600) of claim 22, further comprising:
communicatively pairing the indoor radar device (100) and a control center (360), wherein the indoor radar device (100) and the control center (360) are wirelessly connected via at least one of a frequency division multiplexing manner and a time division multiplexing manner.

24. The indoor event detecting method (600) of any one of claims 22-23, wherein the room (900) is a toilet room and divided into a door-closed detection space (971) and an occupancy detection space (972), the door-closed detection space (971) is adjacent to the door (980), and the occupancy detection space (972) is adjacent to a toilet (990) in the room (900);
wherein the indoor event detecting method (600) further comprises:
detecting whether the door (980) starts to be in the door-closed state operated inside the room (900) by the indoor radar device (100), which comprises detecting whether the detected target moves from the door-closed detection space (971) to the occupancy detection space (972).

25. The indoor event detecting method (600) of any one of claims 22-24, wherein the overtime occupancy condition comprises that an occupancy time value of the detected target in the room (900) is greater than an occupancy time threshold, which is between 3 minutes and 90 minutes.

26. The indoor event detecting method (600) of any one of claims 22-25, wherein performing the vital sign detection comprises at least one of performing a posture detection, performing a heart rate detection and performing a respiration detection;
wherein performing the posture detection comprises detecting whether a detected target stationary condition is satisfied and whether a fall condition is satisfied based on at least five sampling points by the indoor radar device (100), which is a posture detection result;
wherein performing the heart rate detection comprises detecting a heart rate related to a time to determine whether an abnormal heart rate condition is satisfied, which is a heart rate detection result;
wherein performing the respiration detection comprises detecting a respiration rate related to the time to determine whether an abnormal respiration condition is satisfied, which is a respiration detection result;
wherein the indoor event detecting method (600) further comprises:
sending the heart rate and the respiration rate to a control center (360) by the indoor radar device (100) and storing the heart rate and the respiration rate in the control center (360); and
determining whether the dangerous condition is satisfied based on corresponding at least one of the posture detection result, the heart rate detection result and the respiration detection result.
